# EUROPEAN PATENT SPECIFICATION

(11) **EP 1 036 082 B1**
(45) Date of publication and mention of the grant of the patent: **29.05.2002**
(21) Application number: 98957019.7
(22) Date of filing: 04.12.1998
(51) Int. Cl.: C07H 1/00

(54) **ISOLATION OF NUCLEIC ACIDS**
ISOLIERUNG VON NUKLEINSÄUREN
ISOLEMENT D'ACIDES NUCLEIQUES

(30) Priority: 06.12.1997 GB 9725839; 17.07.1998 GB 9815541
(43) Date of publication of application: 20.09.2000
(60) Divisional application: 02003403.9
(73) Proprietor: DNA Research Innovations Limited, Sittingbourne, Kent ME9 8PX (GB)
(72) Inventor: BAKER, Matthew, John, Maidstone Kent ME15 9UJ (GB)
(74) Representative: Kiddle, Simon John
(86) International application number: GB9803602
(87) International publication number: WO9929703

(56) References cited:
- EP-A- 0 301 899
- EP-A- 0 707 077
- EP-A- 0 832 897
- EP-A- 0 897 978
- WO-A-95/27718
- WO-A-96/09116
- WO-A-97/29825
- DATABASE WPI Section Ch, Week 9819 Derwent Publications Ltd., London, GB; Class B04, AN 98-210396 XP002106739 & JP 10 057056 A (ASAHI OPTICAL CO LTD) , 3 March 1998

## Description

The present invention relates to a method for extracting nucleic acids from biological material, particularly blood.

There is a very large demand for DNA analysis for a range of purposes and this has lead to the requirement for quick, safe, high throughput methods for the isolation and purification of DNA and other nucleic acids.

Samples for use for DNA identification or analysis can be taken from a wide range of sources such as biological material such as animal and plant cells, faeces, tissue etc. also samples can be taken from soil, foodstuffs, water etc.

Existing methods for the extraction of DNA include the use of phenol/chloroform, salting out, the use of chaotropic salts and silica resins, the use of affinity resins, ion exchange chromatogtaphy and the use of magnetic beads. Methods are described in US Patents 5057426, 4923978, EP Patents 0512767 Aland EP0515484B and WO 95/13368, WO 97/10331 and WO 96/18731. These patents and patent applications disclose methods of adsorbing nucleic acids on to a solid support and then isolating the nucleic acids. The previously used methods use some type of solvent to isolate the nucleic acids and these solvents are flammable, combustible or toxic.

EP 0 707077A describes the capture and selective release of nucleic acid from a lysate by using a water-soluble, weakly basic polymer to form a precipitate with the polymer The precipitate is then separated and the nucleic acid released from the polymer using extremely high salt conditions, strong base or heating.

Blood is one of the most abundant sample sources for DNA analysis as blood samples are routinely taken for a wide range of reasons. However because of the viscous and proteinaceous nature of blood using known DNA extraction methods it has proved difficult to accomplish using automation due to the difficulties of handling large volumes containing relatively small amounts of DNA. Hitherto nucleic acid extraction has been partially automated only by using hazardous reagents and slow processing times.

I have now devised an improved method for the extraction of nucleic acids and other biomolecules from blood and other biological materials.

Accordingly, the present invention provides a method of extracting nucleic acid from a biological material as set out in claim 1.

The method is particularly useful if the biological material is blood, but the method can be used for a range of applications substances such as Plasmid and vector isolation and plant DNA extraction.

Preferably the cells in the blood are lysed to release nucleic acids and known lysing agents and methods can be used, such as contacting with ionic and non ionic detergents, hypotonic solutions of salts, proteases, chaotropic agents, solvents, using pH changes or heat. A method of lysing cells to isolate nucleic acid is described in WO 96/00228.

When the biological material consists of blood the samples can optionally be diluted with water or other diluent in order to make it easier to manipulate and to process.

Dilutions up to ten times can be used and in general more dilution can be better and it is a feature of the present invention that it allows low dilution of blood to be possible.

The solid phase with which the blood is contacted, can be a formed of a material which has a natural affinity for nucleic acids or it can be formed of a material which has its surface treated with an agent which will cause nucleic acids to bind to it or increase its affinity for nucleic acids. Suitable materials include controlled pore glass, polysaccharide (agarose or cellulose), other types of silica/glass, ceramic materials, porous plastic materials such as porous plastic plugs which in a single moulded part or as an insert in a standard tube, polystyrene beads para magnetic beads etc. The size and porosity is not critical and can vary and be selected for particular applications.

Suitable means for treating the surface of the solid phase or for derivitising it include treating it with a substance which can introduce a charge e.g. a positive charge on the surface or a hydrophilic or hydrophobic surface on the solid phase e.g. hydroxyl groups, nitrate groups, autoreactive groups, dyes and other aromatic compounds.

In a preferred embodiment of the invention the solid phase will cause DNA to be bound to it at one pH in preference to contaminants in the blood sample and will allow the bound nucleic acid to be released when it is contacted with an eluant at a different pH. This system can be used with a solid phase which incorporates histidine or a polyhistidine which will tend to bind nucleic acids at low pH e.g. less than 6 and will then release the bound nucleic acids when the pH is increased e.g. to greater than 8. Alternatively the nucleic acids are bound at substantially neutral pH to an aminated surface and released at very high pH.

In another embodiment of the invention a plastic moulding can incorporate a binding agent e.g. in a well in a plate etc. so that the binding agent is incorporated in the surface, the blood sample is then contacted with the surface so as to cause nucleic acids to be bound to the surface. The blood sample is then removed and the surface treated with an eluting agent to release the bound nucleic acids. When the surface is part of a well in a multi- well plate, the total system can be readily adapted for rapid large scale sampling and extraction techniques.

Binding agents which can be used include charge switchable ion exchange resins using a positively charged solid phase that can be reversed or made neutral by changing the pH above its pKa. e.g. nucleotides, polyamines, imidazole groups and other similar reagents with a suitable pKa value.

Also, nucleic acids can be bound by intercalation using a variety of intercalating compounds incorporated into the solid phase e.g. Actinomycin D, Ethidium Bromide etc.

In a further embodiment of the invention a plastic surface can be modified to include functional groups. The plastic can be any plastic used for containing samples e.g. polypropylene. The functional groups can be positively or negatively charged so as to bind the nucleic acids in the correct buffer solution.

Alternatively the functional groups can be chemical groups capable of covalent coupling to other ligands or polymers.

When the plastic is used in a plastic moulding e.g. in a well in a plate, or as a Polymerase Chain Reaction (PCR) tube, the surface characteristics of the plastic can be suitably modified for use in the present invention by including or adding the appropriate chemicals in the moulding compound e.g. as in an injection moulding compound.

When this is used in a PCR tube or in a deep well plate the tubes or wells can be used to isolate and immobilise small quantities of DNA or RNA generating a pure template for subsequent PCR or other genetic analysis and manipulation.

When the plastic is polypropylene e.g. it is in the form of a thin walled PCR tube the polypropylene surface can be modified by oxidising the surface with an oxidising agent such as potassium permanganate and sulphuric acid to create a carboxylated surface (COOH groups). This tube can then be used to improve the isolation of DNA from solutions or from crude samples e.g. blood. By adjusting the pH, di-electric constant, solubility or ionic strength the DNA or RNA can be immobilised on the walls of the tube, washed free of contaminants, ready for PCR or other analytical techniques.

The carboxy groups can be further modified by covalently coupling an anionic group such as imidazole or polyhistidine or any strong or weak ion exchanger, to allow binding of nucleic acids by a charge interaction. This tube could then be used to improve the isolation of DNA from solutions or from crude samples e.g. of blood. Again by adjusting the pH, di-electric constant, or ionic strength the DNA or RNA can be immobilised on the walls of the tube, washed free of contaminants, ready for PCR or other analytical techniques.

The nucleic acids can be eluted with in a low salt buffer so that it is ready for PCR or other analysis.

The solid phase can be contacted with a blood sample by mixing with the solid phase in a mixing/ stirring device, by passing the blood sample over the solid phase or the solid phase can be paramagnetic and manipulated by a magnetic field. Although the invention is particularly suitable for the separation or isolation of nucleic acids from blood it can be used with a range of biomolecules particularly those that require removal of cell wall debris or insoluble particles.

In a preferred embodiment of the invention the solid phase is in granular form in a column and the blood sample is drawn up through the column by means of a pressure differential being applied through the column, the blood sample is drawn up with air and the granular solid material can become fluidised thus increasing the mixing and contacting rates and minimising clogging.

The method of the invention is suitable for use in a multi-well format when a series of extractions from different samples can take place substantially simultaneously and this will facilitate the automation of the extraction process allowing rapid high throughput extraction to take place and to allow combinational chemistry to be performed. This will enable there to be a high throughput in a standard well array e.g. an eight by twelve array so that a large number of sample types can be treated automatically at the same time.

The invention is described in the Example.

### Example 1

### Extraction of Nucleic Acids from Whole Blood

A charge switchable ion-exchanger was prepared by covalently coupling polyhistidine to 100 (m glass beads using glutaldehyde by mixing 1 gram of the aminated glass beads with 0.01 %(v/v) glutaldehyde in O.1M sodium bicarbonate at pH8 containing 20mg polyhistidine. After overnight incubation the beads were washed exhaustively to remove non-covalently bound material and stored in 10mM MES, pH5 containing 0.1 % (v/v) Tween 20.

About 300mg of the 100(m derivitised glass beads were added to a lml plastic column enclosed at both ends.

A blood sample was incubated with an equal volume of 10mM MES pH5, containing 1% Tween 20, proteases (200(g/ml) and 1 mM EDTA. After digestion is complete the blood was sucked up the column containing the glass beads and the DNA became immobilised allowing the contaminating proteins to pass through to waste.

The glass beads containing the immobilised DNA were washed with a buffer comprising 10mM MES pH5, containing 1% Tween 20, and 1mM EDTA and this was repeated until the wash solution was colourless.

After washing, the beads were dried with air and DNA eluted with a small quantity of 10mM Tris HCI, pH 8.5 and collected in a sterile tube ready for analysis. Thus the DNA were separated from the blood.

For different biomolecules, the buffer etc. can be suitably modified.

### Example 2

One gram of carboxylated paramagnetic beads were washed in 50mM Imidazole buffer pH6 and then mixed with 100mg of polyhistidine in 50ml of 50mM Imidazole buffer pH 6. A chemical coupling agent was added (EDC) at a final concentration of 5mg per ml and mixed overnight. The beads were washed in water, 0.5M sodium chloride, 1% Tween 20, 100mM Tris HCl pH 8 and stored in 10mM MES, 0.1% Tween 20 pH 5.

To extract DNA from blood, 1mg of beads were mixed with blood diluted in 10% Tween 20 with 25mM MES, 1mM EDTA pH 5. The beads were separated with a magnet and washed by resuspending in 1mM MES, 0.1% Tween 20. To elute the DNA the beads were resuspended in 10mM Tris HCl pH 8.5 and separated with a magnet leaving the DNA in solution.

## Claims

1. A method of extracting nucleic acid from a biological material containing nucleic acid, which method comprises:
bringing the biological material into contact with a solid phase at a first pH at which the solid phase has a positive charge so that the nucleic acid binds to the solid phase in preference to contaminants in the biological material;
releasing the nucleic acid at a second, higher pH above the pKa of the solid phase to reverse or neutralise the positive charge to release the bound nucleic acid from the solid phase;
wherein the solid phase is controlled pore glass, a polysaccharide, a ceramic material, a porous plastic material, a plastic material, polystyrene beads, paramagnetic beads, or a porous plastic plug which is a single moulded part or is an insert for a container; and,
wherein the nucleic acid is released using a low salt buffer.

2. The method of claim 1, wherein the plastic material is in the form of a pipette tip, a well plate or deep well plate, or a PCR tube.

3. The method of claim 1 or claim 2, wherein the eluted nucleic acid is ready for analysis or amplification using PCR without the need for further purification.

4. The method of any one of claims 1 to 3, wherein the low salt buffer is 10mM Tris HCl at pH 8.5.

5. The method of any one of preceding claims, wherein the nucleic acid binds to the solid phase at a pH of less than 6 and is released from the solid phase at a pH of greater than 8.

6. The method of any one of the preceding claims, wherein the nucleic acid binds to the solid phase at a substantially neutral pH and is released from the solid phase at a pH of greater than 10.

7. The method of any one of the preceding claims, wherein the solid phase is formed of a material which has a natural affinity for nucleic acids.

8. The method of any one of the preceding claims, wherein the solid phase is formed of a material which has its surface treated with an agent which introduces a positive charge to cause nucleic acids to bind to it or to increase its affinity for nucleic acids.

9. The method of any one of the preceding claims, wherein the solid phase capable of binding nucleic acid is a charge switchable ion exchange resin having a positive charge that can be reversed or neutralised by changing the pH above its pKa.

10. The method of claim 9, wherein the ion exchange resin comprises a nucleotide, a polyamine or a compound containing an imidazole moiety.

11. The method of claim 8, wherein the surface of the solid material is treated with histidine or a polyhistidine.

12. The method of any one of the preceding claims,
wherein the solid phase comprises a plastic surface modified to include functional groups.

13. The method of claim 11 or claim 12, wherein the plastic material is polypropylene.

14. The method of claim 13, wherein the polypropylene surface is modified by oxidising the surface with an oxidising agent to create a carboxylated surface.

15. The method as claim 14, wherein the carboxyl groups are further modified by covalently coupling an anion exchange group to allow binding of nucleic acids by a charge interaction.

16. The method of claim 15, wherein the anionic exchange group is imidazole, polyhistidine or a strong or weak ion exchanger.

17. The method of any one of the preceding claims, wherein the solid phase is a PCR tube or a well plate which is used to isolate and immobilise small quantities of nucleic acid, thereby generating a template for subsequent PCR or other genetic analysis and manipulation.

18. The method of any one of the preceding claims, the solid phase is a multi-well plate thereby allowing a series of extractions of nucleic acid from different samples to take place substantially simultaneously.

19. The method of any one of the preceding claims, wherein the solid phase is contacted with a blood sample by mixing with the solid material in a mixing/stirring device, by passing the blood sample over the solid phase or the solid phase is manipulated on a magnetisable support.

20. The method of any one of the preceding claims, wherein the solid phase is in a column and the blood sample is drawn up through the column by means of a pressure differential being applied through the column.

21. The method of claim 20, wherein the blood sample is drawn up with air and the solid phase becomes fluidised.

22. The method of any one of the preceding claims, wherein the sample is a blood sample.

23. The method of claim 22, wherein cells in the blood are lysed to release the nucleic acid.

24. The method of claims 23, wherein the cells are lysed by contacting with an ionic or non ionic detergent, hypotonic solutions of a salt, a protease, a chaotropic agents or by use of pH changes or heat.

25. The method of claim 23 or claim 24, comprising diluting the blood sample with water or other diluents so that it is easier to manipulate and to pour.

## Patentansprüche

1. Verfahren zum Extrahieren von Nucleinsäure aus einem Nucleinsäure-hältigen biologischen Material, wobei das Verfahren Folgendes umfasst:
das In-Kontakt-Bringen des biologischen Materials mit einer Festphase bei einem ersten pH, bei dem die Festphase eine positive Ladung aufweist, so dass sich die Nucleinsäure gegenüber Verunreinigungen bevorzugt im biologischen Material an die Festphase bindet;
das Freisetzen der Nucleinsäure bei einem zweiten, höheren pH, der über dem pKa der Festphase liegt, um die positive Ladung umzupolen oder zu neutralisieren, um die gebundene Nucleinsäure aus der Festphase freizusetzen;
worin die Festphase aus einem Glas mit regulierter Porengröße, einem Polysaccharid, einem Keramikmaterial, einem porösen Kunststoffmaterial, einem Kunststoffmaterial, Polystyrolperlen, paramagnetischen Perlen oder einem porösen Kunststoff-Pfropfen besteht, der ein einzelner Formteil oder ein Einsatz für einen Behälter ist; und
worin die Nucleinsäure unter Einsatz eines salzarmen Puffers freigesetzt wird.

2. Verfahren nach Anspruch 1, worin das Kunststoffmaterial in Form einer Pipettenspitze, einer Napfplatte oder einer Tiefnapfplatte oder in Form eines PCR-Röhrchens vorliegt.

3. Verfahren nach Anspruch 1 oder 2, worin die eluierte Nucleinsäure zur Analyse oder Amplifizierung unter Einsatz von PCR bereit ist, ohne dass weitere Reinigung erforderlich ist.

4. Verfahren nach einem der Ansprüche 1 bis 3, worin der salzarme Puffer 10 mM Tris-HCl mit pH 8,5 ist.

5. Verfahren nach einem der vorangegangenen Ansprüche, worin sich die Nucleinsäure bei einem pH unter 6 an die Festphase bindet und bei einem pH über 8 von der Festphase freigesetzt wird.

6. Verfahren nach einem der vorangegangenen Ansprüche, worin sich die Nucleinsäure bei einem im Wesentlichen neutralen pH an die Festphase bindet und bei einem pH über 10 von der Festphase freigesetzt wird.

7. Verfahren nach einem der vorangegangenen Ansprüche, worin die Festphase aus einem Material besteht, das eine natürliche Affinität für Nucleinsäuren aufweist.

8. Verfahren nach einem der vorangegangenen Ansprüche, worin die Festphase aus einem Material besteht, dessen Oberfläche mit einem Mittel behandelt ist, das eine positive Ladung einführt, um zu bewirken, dass sich Nucleinsäuren daran binden, oder um seine Affinität für Nucleinsäuren zu erhöhen.

9. Verfahren nach einem der vorangegangenen Ansprüche, worin die zur Bindung von Nucleinsäure fähige Festphase einen lonenaustauschharz mit veränderbarer Ladung ist, das eine positive Ladung aufweist, die durch Änderung des pH über seinen pKa umgepolt oder neutralisiert werden kann.

10. Verfahren nach Anspruch 9, worin das lonenaustauschharz ein Nucleotid, ein Polyamin oder eine Verbindung umfasst, die eine Imidazolgruppe enthält.

11. Verfahren nach Anspruch 8, worin die Oberfläche des festen Materials mit Histidin oder einem Polyhistidin behandelt ist.

12. Verfahren nach einem der vorangegangenen Ansprüche, worin die Festphase eine Kunststoffoberfläche umfasst, die so modifiziert ist, dass sie funktionelle Gruppen enthält.

13. Verfahren nach Anspruch 11 oder 12, worin das Kunststoffmaterial Polypropylen ist.

14. Verfahren nach Anspruch 13, worin die Polypropylenoberfläche durch Oxidieren der Oberfläche mit einem Oxidationsmittel modifiziert wird, um ein carboxylierte Oberfläche zu schaffen.

15. Verfahren nach Anspruch 14, worin die Carboxylgruppen weiters durch kovalente Bindung einer Anionenaustauschgruppe modifiziert werden, um die Bindung von Nucleinsäuren durch Ladungswechselwirkung zu ermöglichen.

16. Verfahren nach Anspruch 15, worin die Anionenaustauschgruppe Imidazol, Polyhistidin oder ein starker oder schwacher lonenaustauscher ist.

17. Verfahren nach einem der vorangegangenen Ansprüche, worin die Festphase ein PCR-Röhrchen oder eine Napfplatte ist, die verwendet wird, um geringe Mengen an Nucleinsäure zu isolieren und zu immobilisieren, wodurch ein Templat für nachfolgende PCR oder andere genetische Analyse und Manipulation erzeugt wird.

18. Verfahren nach einem der vorangegangenen Ansprüche, worin die Festphase eine Mehrnapfplatte ist, wodurch es ermöglicht wird, dass eine Reihe von Nucleinsäure-extraktionen aus verschiedenen Proben im Wesentlichen gleichzeitig erfolgt.

19. Verfahren nach einem der vorangegangenen Ansprüche, worin die Festphase mit einer Blutprobe in Kontakt gebracht wird, indem sie in einer Misch/Rühr-Vorrichtung mit dem festen Material vermischt wird, indem die Blutprobe über die Festphase geschickt wird oder indem die Festphase auf einem magnetisierbaren Träger manipuliert wird.

20. Verfahren nach einem der vorangegangenen Ansprüche, worin die Festphase eine Säule ist und die Blutprobe durch die Säule aufgezogen wird, indem ein Druckunterschied durch die Säule angelegt wird.

21. Verfahren nach Anspruch 20, worin die Blutprobe mit Luft aufgezogen wird und die Festphase fließfähig gemacht wird.

22. Verfahren nach einem der vorangegangenen Ansprüche, worin die Probe eine Blutprobe ist.

23. Verfahren nach Anspruch 22, worin Zellen im Blut lysiert werden, um die Nucleinsäure freizusetzen.

24. Verfahren nach Anspruch 23, worin die Zellen lysiert werden, indem sie mit einem ionischen oder nichtionischen Detergens, hypotonischen Lösungen eines Salzes, einer Protease oder einem chaotropen Mittel in Kontakt gebracht werden, oder unter Einsatz von pH-Änderungen oder Wärme.

25. Verfahren nach Anspruch 23 oder 24, umfassend das Verdünnen der Blutprobe mit Wasser oder anderen Verdünnern, so dass sie leichter manipulierbar und fließfähig ist.

## Revendications

1. Méthode d'extraction d'acide nucléique d'une matière biologique contenant de l'acide nucléique, laquelle méthode consiste à :
mettre la matière biologique en contact avec une phase solide à un premier pH auquel la phase solide a une charge positive de façon que l'acide nucléique se lie à la phase solide préférentiellement à des contaminants dans la matière biologique.
libérer de l'acide nucléique à un second pH plus élevé, au-dessus de pKa de la phase solide pour inverser ou neutraliser la charge positive pour libérer l'acide nucléique lié de la phase solide;
où la phase solide est un verre à pores contrôlées, un polysaccharide, un matière céramique, une matière plastique poreuse, une matière plastique, des perles de polystyrène, des perles paramagnétiques, ou un bouchon en plastique poreux qui est une pièce moulée individuelle où est un insert pour un conteneur; et
où l'acide nucléique est libéré en utilisant un tampon à faible teneur en sel.

2. Méthode de la revendication 1, où la matière plastique est sous la forme d'une pointe de pipette, d'une plaque à puits ou d'une plaque à puits profond ou un tube de PCR.

3. Méthode de la revendication 1 ou de la revendication 2, où l'acide nucléique élue est prêt pour l'analyse ou l'amplification en utilisant PCR sans la nécessité d'une plus ample purification.

4. Méthode selon l'une quelconque des revendications 1 à 3, où le tampon à faible teneur en sel est Tris HCl 10mM à pH 8,5.

5. Méthode selon l'une quelconque des revendications précédentes, où l'acide nucléique se lie à la phase solide à un pH de moins de 6 et est libéré de la phase solide à un pH de plus de 8.

6. Méthode selon l'une quelconque des revendications précédentes, où l'acide nucléique se lie à la phase solide à un pH sensiblement neutre et est libéré de la phase solide à un pH de plus de 10.

7. Méthode selon l'une quelconque des revendications précédentes, où la phase solide est formée d'une matière qui a une affinité naturelle pour les acides nucléiques.

8. Méthode selon l'une quelconque des revendications précédentes, où la phase solide est formée d'une matière qui a sa surface traitée avec un agent qui introduit une charge positive pour forcer les acides nucléiques à se lier à elle ou pour augmenter son affinité pour les acides nucléiques.

9. Méthode selon l'une quelconque des revendications précédentes, où la phase solide capable de lier l'acide nucléique est une résine échangeuse d'ions à charge changeable ayant une charge positive qui peut être inversée ou neutralisée en changeant le pH au-dessus de son pKa.

10. Méthode de la revendication 9, où la résine échangeuse d'ions comprend un nucléotide, une polyamine ou un composé contenant une fraction d'imidazole.

11. Méthode de la revendication 8, où la surface de la matière solide est traitée avec de l'histidine ou une polyhistidine.

12. Méthode selon l'une quelconque des revendications précédentes où la phase solide comprend une surface de plastique modifiée pour inclure des groupes fonctionnels.

13. Méthode de la revendication 11 ou de la revendication 12, où la matière plastique est du polypropylène.

14. Méthode de la revendication 13, où la surface de polypropylène est modifiée par oxydation de la surface avec un agent oxydant pour créer une surface carboxylée.

15. Méthode de la revendication 14, où les groupes carboxyles sont de plus modifiés par couplage covalent d'un groupe échangeur d'anions pour permettre la liaison des acides nucléiques par une interaction de charge.

16. Méthode de la revendication 15, où le groupe échangeur d'anions est imidazole, polyhistidine ou un échangeur d'ions faible ou fort.

17. Méthode selon l'une quelconque des revendications précédentes, où la phase solide est un tube de PCR ou une plaque à puits que l'on utilise pour isoler et immobiliser des petites quantités d'acide nucléique afin de générer ainsi une matrice pour PCR subséquente ou autre analyse et manipulation génétique.

18. Méthode selon l'une quelconque des revendications précédentes, la phase solide est une plaque multi-puits pour ainsi permettre à une série d'extraction de l'acide nucléique de différents échantillons d'avoir lieu sensiblement simultanément.

19. Méthode selon l'une quelconque des revendications précédentes, où la phase solide est mise en contact avec un échantillon de sang par mélange avec la matière solide dans un dispositif de mélange/agitation, par passage de l'échantillon de sang sur la phase solide ou bien la phase solide est manipulée sur un support magnétisable.

20. Méthode de l'une quelconque des revendications précédentes où la phase solide est dans une colonne et l'échantillon de sang est attiré à travers la colonne au moyen d'une différence de pression qui est appliquée à travers la colonne.

21. Méthode de la revendication 20, où l'échantillon de sang est attiré avec de l'air et la phase solide se trouve fluidisée.

22. Méthode selon l'une quelconque des revendications précédentes, où l'échantillon est un échantillon de sang.

23. Méthode de la revendication 22, où les cellules dans le sang sont lysées pour libérer l'acide nucléique.

24. Méthode de la revendication 23, où les cellules sont lysées par mise en contact avec un détergent ionique ou non ionique, des solutions hypotoniques d'un sel, une protéase, des agents chaotropes ou par utilisation de changements de pH ou de chaleur.

25. Méthode de la revendication 23 où de la revendication 24, consistant à diluer l'échantillon de sang avec de l'eau ou d'autres diluants pour qu'il soit plus facile à manipuler et à verser.
